# EUROPEAN PATENT APPLICATION

(11) **EP 1 878 442 A1**
(43) Date of publication of application: **16.01.2008**
(21) Application number: 06732363.4
(22) Date of filing: 26.04.2006
(51) Int. Cl.: A61K 41/00, A61K 31/409, A61P 35/00, A61P 35/02

(54) **METHOD FOR TREATING BODY FLUID**

(30) Priority: 26.04.2005 JP 2005128457
(71) Applicant: HAMAMATSU PHOTONICS K.K., Hamamatsu-shi, Shizuoka-ken 435-8558 (JP)
(72) Inventor: TAKEI, Hiroshi, Hamamatsu-shi, Shizuoka 4358558 (JP); MINEMATSU, Sumio, Hamamatsu-shi, Shizuoka 4358558 (JP); MIYAKI, Sueo, Hamamatsu-shi, Shizuoka 4358558 (JP); FUJISAKA, Shinichi, Hamamatsu-shi, Shizuoka 4358558 (JP); SAKATA, Isao, Okayama-shi, Okayama 7000086 (JP); NAKAE, Yoshinori, Okayama-shi, Okayama 7038244 (JP)
(74) Representative: Frost, Alex John
(86) International application number: PCT/JP2006/308757
(87) International publication number: WO 2006/115273

(57) **Abstract**

The invention provides a body fluid treatment method for selective *ex vivo* killing of malignant lymphoma cells, leukemia cells or activated macrophages in a body fluid, the body fluid treatment method comprising: an addition step wherein a compound represented by formula (I) below is added to a body fluid containing malignant lymphoma cells, leukemia cells or activated macrophages that has been removed from the body, to yield an addition mixture; and an excitation step wherein the addition mixture is irradiated with excitation light to excite the compound. According to the invention, there is provided a method for selective *ex vivo* killing of malignant lymphoma cells, leukemia cells or activated macrophages in a body fluid while avoiding adverse effects on normal cells.

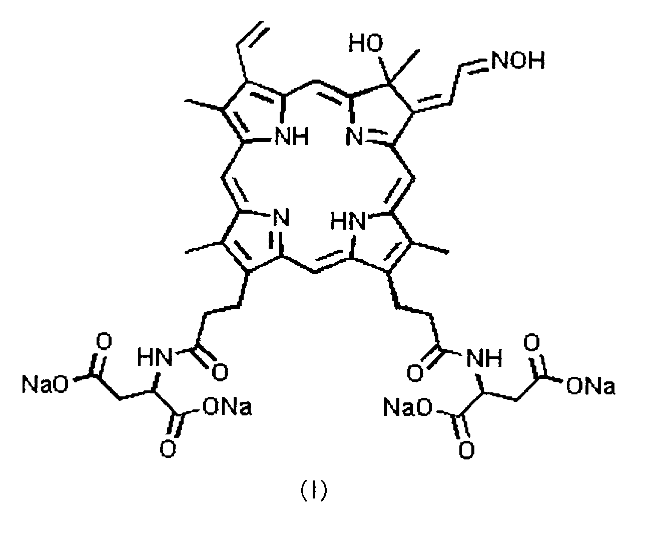

## Description

### Technical Field

The present invention relates to a body fluid treatment method.

### Background Art

Extracorporeal photochemotherapy, or photopheresis, is known as a method for *ex vivo* killing of malignant lymphoma cells in a body fluid. Conventional extracorporeal photochemotherapy entails treating extracorporeally circulated blood with 8-methoxypsoralen (8-MOP), irradiating it with ultraviolet (UVA) to kill the malignant lymphoma cells, and then returning the blood into the body, and it is currently used for treatment of cutaneous T cell lymphoma (Non-patent document 1).
Non-patent document 1: Haematologica 1999; 84:237-241

### Disclosure of the Invention

### Problem to be Solved by the Invention

However, since UVA (wavelength: 320 nm to 400 nm) is used for the method mentioned above, it has not been possible to avoid the adverse effects of light irradiation on normal cells (especially their nuclei).

It is an object of the present invention to provide a method for killing malignant lymphoma cells in a body fluid *ex vivo* while avoiding adverse effects on normal cells.

### Means for Solving the Problem

In order to achieve the object stated above, the invention provides a body fluid treatment method for selective *ex vivo* killing of malignant lymphoma cells, leukemia cells or activated macrophages in a body fluid, the body fluid treatment method comprising: an addition step wherein a compound represented by formula (I) or (II) below (hereinafter also referred to as "ATX-S10·Na", while the compound represented by formula (II) is also referred to as "ATX-S10·Na(II)") is added to a body fluid containing malignant lymphoma cells, leukemia cells or activated macrophages that has been removed from the body, to yield an addition mixture; and an excitation step wherein the addition mixture is irradiated with excitation light to excite the compound represented by formula (I) or (II).

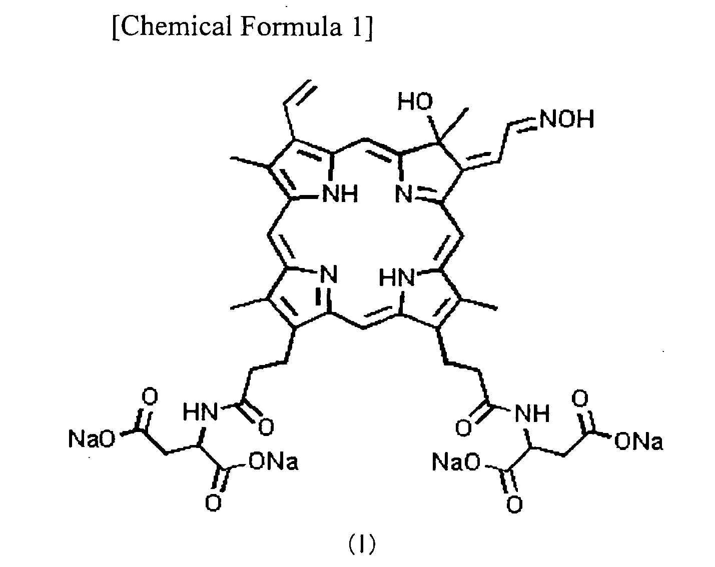

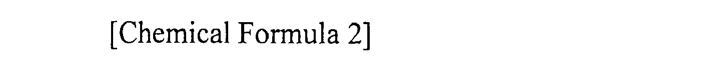

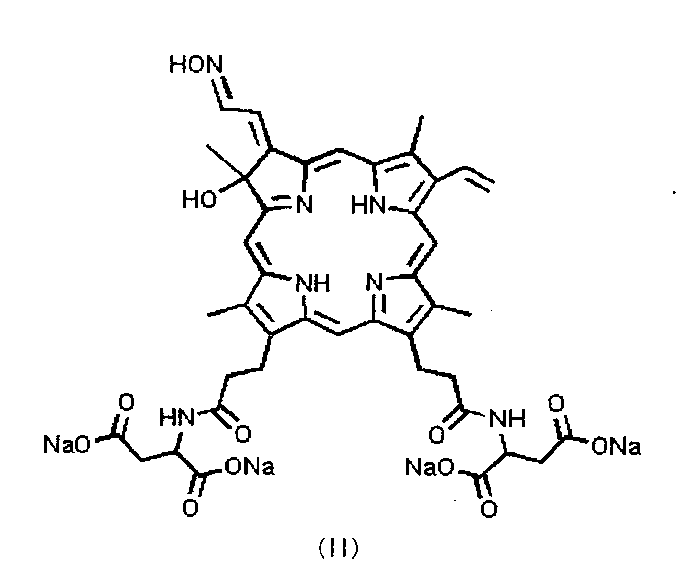

ATX-S10·Na is taken up by malignant lymphoma cells, leukemia cells and activated macrophages in a body fluid, while almost no ATX-S10·Na is taken up by erythrocytes and platelets. Consequently, addition of ATX-S10·Na to a body fluid containing malignant lymphoma cells, leukemia cells or activated macrophages results in selective uptake of ATX-S10·Na into the aforementioned types of cells (malignant lymphoma cells, leukemia cells and activated macrophages). Irradiating such a body fluid with excitation light to excite the ATX-S10·Na causes death of the cells (malignant lymphoma cells, leukemia cells and activated macrophages) that have taken up the ATX-S10·Na. In other words, this body fluid treatment method is a method for selective *ex vivo* killing of malignant lymphoma cells, leukemia cells or activated macrophages in a body fluid. Here, "killing" means killing of at least part of the population of malignant lymphoma cells, leukemia cells or activated macrophages in the body fluid, and does not necessarily refer to killing of all the population.

ATX-S10·Na is excited by irradiation of light with a wavelength of 400 nm to 450 nm or approximately 670 nm (650 nm to 700 nm). That is, light in the visible light range, which has a longer wavelength than UVA, is used for this body fluid treatment method. Consequently, the light irradiation causes virtually no adverse effects on normal cells (especially their nuclei) or the apparatus materials. Moreover, since the light has high substance permeability, a sufficient amount of the light can reach the target cells.

When a body fluid treated by the body fluid treatment method described above is returned to the body, the normal cells are restored to the body essentially without suffering any adverse effects. Thus, if a body fluid from a patient with malignant lymphoma, leukemia or autoimmune disease (ulcerative colitis, Crohn's disease, rheumatoid arthritis or the like) is treated by the body fluid treatment method and returned to the patient's body, the disease is treated essentially without any adverse effects on normal tissues or cells.

In this body fluid treatment method, it is preferred that prior to the addition step, there is performed a separation step in which the leukocyte fraction is separated from the body fluid, and that in the addition step, the ATX-S10·Na is added to the leukocyte fraction. This will allow the non-leukocyte fraction (erythrocytes, platelets, etc.) to be promptly returned to the patient's body to effectively prevent hypotension, anemia, etc. in the patient. Addition of ATX-S10·Na to the leukocyte fraction is included in the concept of addition of ATX-S10·Na to the "body fluid containing malignant lymphoma cells, leukemia cells or activated macrophages".

If a body fluid from a patient with malignant lymphoma or leukemia is treated by the body fluid treatment method described above and the treated body fluid is returned to the patient's body, proliferation of the malignant lymphoma cells or leukemia cells in the body is markedly inhibited. That is, the killed malignant lymphoma cells or leukemia cells in the treated body fluid act as a vaccine for malignant lymphoma or leukemia. This is presumably due to the fact that necrotic cells and apoptotic cells are present in the treated body fluid in good proportion, and that in the body, these dead cells activate immunocytes, particularly T lymphocytes, which are specific for cells of the same kind as the dead cells.

Thus, a treated body fluid containing a vaccine for malignant lymphoma or leukemia can be obtained by addition of ATX-S10·Na to a body fluid containing malignant lymphoma cells or leukemia cells that has been removed from the body, and irradiation with excitation light. The vaccine in the treated body fluid is a tailor-made vaccine that induces immunity highly specific to the patient's own malignant lymphoma cells or leukemia cells, and it makes it possible to treat malignant lymphoma or leukemia, and to prevent relapse or metastasis after treatment.

Using the body fluid treatment method described above, it is possible to conveniently and rapidly produce a vaccine for malignant lymphoma or leukemia without performing complicated manipulations such as fixation of the target malignant lymphoma cells or leukemia cells with formalin or the like.

The use of ATX-S10·Na for *in vivo* administration is described in Japanese Patent Publication No. 3613599 and Japanese Patent Publication No. 3191223, but the present inventors have discovered for the first time that ATX-S10·Na can be used for *ex vivo* body fluid treatment, especially for body fluid treatment for the purpose of vaccination.

### Effects of the Invention

According to the invention, there is provided a method for selective *ex vivo* killing of malignant lymphoma cells, leukemia cells or activated macrophages in a body fluid while avoiding adverse effects on normal cells. There is also provided a vaccine most suitable for an individual malignant lymphoma or leukemia patient.

### Brief Description of the Drawings

Fig. 1 is a bar graph showing ATX-S10·Na(II) uptake by different types of cells immediately after addition of ATX-S10·Na(II) to the cell suspensions.
Fig. 2 is a bar graph showing ATX-S10·Na(II) uptake by different types of cells after addition of ATX-S10·Na(II) to the cell suspensions and incubation at 37°C for 1 hour.
Fig. 3 is a bar graph showing ATX-S10·Na(II) uptake by different types of cells after addition of ATX-S10·Na(II) to the cell suspensions and standing at 4°C for 1 hour.
Fig. 4 is a bar graph showing ATX-S10·Na(II) uptake by different types of cells after addition of ATX-S10·Na(II) to the cell suspensions and incubation at 37°C for 3 hours.
Fig. 5 is a bar graph showing ATX-S10·Na(II) uptake by different types of cells after addition of ATX-S10·Na(II) to the cell suspensions and standing at 4°C for 3 hours.
Fig. 6 is a bar graph showing ATX-S10·Na(II) uptake by different types of cells after addition of ATX-S10·Na(II) to the cell suspensions and incubation at 37°C for 20 hours.
Fig. 7 is a bar graph showing ATX-S10·Na(II) uptake by different types of cells after addition of ATX-S10·Na(II) to the cell suspensions and standing at 4°C for 20 hours.
Fig. 8 is a bar graph showing ATX-S10·Na(II) uptake by different types of cells after addition of ATX-S10·Na(II) to the cell suspensions and incubation at 37°C for 44 hours.
Fig. 9 is a bar graph showing ATX-S10·Na(II) uptake by different types of cells after addition of ATX-S10·Na(II) to the cell suspensions and standing at 4°C for 44 hours.

### Best Modes for Carrying Out the Invention

Preferred embodiments of the invention will now be explained.

The body fluid treatment method of the invention comprises the aforementioned addition step and excitation step.

In the addition step, ATX-S10·Na is added to the body fluid that has been extracted from the body. ATX-S10·Na can be produced by the method described in Japanese Patent Publication No. 3613599.

The body fluid may be blood, lymph or bone marrow fluid. For treatment of blood, it is preferred to add an anticoagulant (heparin, citric acid, ethylenediaminetetraacetic acid (EDTA) or the like) once the blood has been removed from the body, to prevent blood coagulation.

The body fluid is removed from a malignant lymphoma, leukemia or autoimmune disease patient (human or animal), and contains malignant lymphoma cells, leukemia cells or activated macrophages. The malignant lymphoma cells may be derived from either Hodgkin's disease or non-Hodgkin's lymphoma, and may be either B lymphocyte lineage cells or T lymphocyte lineage cells. The leukemia cells may be derived from acute myeloid leukemia, chronic myeloid leukemia, acute lymphocytic leukemia or chronic lymphocytic leukemia, and examples thereof include myeloblasts, promyelocytes, monocytes, B lymphocytes and T lymphocytes.

ATX-S10·Na may be added to the body fluid in a form dissolved in an appropriate solvent, or it may be added in the solid state to the body fluid. The body fluid to which ATX-S10·Na has been added is preferably incubated for more than a certain period of time (for example, 2 hours) so that the ATX-S10·Na is fully taken up by the malignant lymphoma cells, leukemia cells or activated macrophages. Since ATX-S10·Na is highly water-soluble, the solvent is preferably saline, PBS (phosphate-buffered saline) or the like.

Separation of the leukocyte fraction from the body fluid can be accomplished by centrifugation based on differences in specific gravity, for example. The obtained leukocyte fraction is preferably suspended in a solvent such as saline, PBS or the like.

The leukocyte fraction needs to contain malignant lymphoma cells, leukemia cells or activated macrophages, while other cells, i.e. normal cells (for example, neutrophils), are preferably separated from the leukocyte fraction. Separation of normal cells makes it possible to more effectively avoid the influences of ATX-S 10·Na on normal cells when ATX-S10·Na is added to the leukocyte fraction.

If the leukocyte fraction is separated from the body fluid, ATX-S10·Na is added to the leukocyte fraction. ATX-S10·Na may be added to the leukocyte fraction in a form dissolved in an appropriate solvent (saline, PBS or the like), or it may be added in the solid state to the leukocyte fraction that has been suspended in an appropriate solvent (saline, PBS or the like). The leukocyte fraction to which ATX-S10·Na has been added is preferably incubated for more than a certain period of time (for example, 2 hours) so that the ATX-S 10·Na is fully taken up by the malignant lymphoma cells, leukemia cells or activated macrophages.

In the excitation step, excitation light is irradiated onto the body fluid or leukocyte fraction containing the added ATX-S10·Na. The excitation light is light with a wavelength of 400 nm to 450 nm, or approximately 670 nm (650 nm to 700 nm), but the wavelength of light used is preferably about 670 nm (650 nm to 700 nm). Light with a longer wavelength has higher substance permeability. The range of 650 nm to 750 nm is the wavelength range with the smallest effect of light absorption on components of the body. If the body fluid is treated without separating the leukocyte fraction, light of 400 nm to 450 nm is possibly absorbed by erythrocytes (hemoglobin) in the body fluid.

The irradiation dose (irradiation energy density) can be appropriately adjusted depending on the amount of ATX-S10·Na taken up by each type of cell in the body fluid or leukocyte fraction. For example, when normal neutrophils are contained in the body fluid or leukocyte fraction, ATX-S10·Na is also possibly taken up into the normal neutrophils. However, since the amount of ATX-S10·Na taken up into neutrophils is not as great as into malignant lymphoma cells, leukemia cells or activated macrophages, it is possible to selectively kill malignant lymphoma cells, leukemia cells or activated macrophages by adjusting the irradiation dose. The irradiation dose is generally preferred to be 1 J/cm² to 50 J/cm².

The excitation light source is one that emits light with a wavelength of 400 nm to 450 nm or approximately 670 nm (650 nm to 700 nm), preferably light of approximately 670 nm (650 nm to 700 nm), and examples thereof include lamps (xenon lamps, etc.), light emitting diodes (LED), laser diodes and the like. When the light source is one that further emits light with a wavelength other than the range above, it is used in combination with a filter that allows the light of the aforementioned wavelength range to be extracted.

The body fluid treatment method of the invention preferably comprises, after the excitation step, a removal step in which the ATX-S10·Na is removed from the body fluid. Removal of the ATX-S10·Na from the body fluid makes it possible to effectively avoid the adverse effects of ATX-S10·Na on normal tissues and cells in the body when the treated body fluid is returned to the body. Removal of the ATX-S10·Na can be accomplished, for example, by passing the body fluid through a column comprising a material that adsorbs ATX-S10·Na (activated carbon or the like).

In the removal step, it is preferred that it is examined whether or not the unremoved ATX-S10·Na exceeds a predetermined amount, and that if the residual ATX-S10·Na is above the predetermined amount, the removal operation is performed again. For example, if ATX-S10·Na excitation light is irradiated onto the body fluid or leukocyte fraction and fluorescence with an intensity exceeding a predetermined value is detected with a fluorescence detector, it can be judged that ATX-S10·Na remains in an amount exceeding the predetermined amount.

The body fluid treatment method of the invention is preferably carried out at a constant temperature of between 25°C and 36°C.

In order to obtain a .treated body fluid containing the vaccine, a body fluid containing malignant lymphoma cells or leukemia cells is treated by the body fluid treatment method described above. In this case, the body fluid treatment method may comprise, after the excitation step (or after the removal step if a removal step is performed), a centrifugation step in which the body fluid or leukocyte fraction is centrifuged and the supernatant is collected. If a centrifugation step is performed, the vaccine is contained in the obtained supernatant.

When the body fluid treatment method described above is used to obtain a treated body fluid containing the vaccine, the body fluid treatment method is preferably one wherein malignant lymphoma cells or leukemia cells are separated from the body fluid or leukocyte fraction prior to the addition step, and then in the addition step, the malignant lymphoma cells or leukemia cells are cultured in medium to which ATX-S10·Na has been added. Culturing of the malignant lymphoma cells or leukemia cells in medium to which ATX-S10·Na has been added is included in the concept of addition of ATX-S10·Na to the "body fluid containing malignant lymphoma cells, leukemia cells or activated macrophages".

The vaccine-containing treated body fluid may be administered to a malignant lymphoma or leukemia patient (human or animal) directly without isolation of the vaccine. When used for a human, it may be administered by intravenous injection, intradermal injection or the like. The number of administrations is preferably 1 to 4.

The treated body fluid containing the malignant lymphoma or leukemia vaccine can be obtained in the following manner, for example. Specifically, 1 × 10⁷ malignant lymphoma cells or leukemia cells separated from the body fluid or leukocyte fraction are plated in each well of a microplate, and ATX-S10·Na(II)-containing medium is added prior to culturing for 24 hours. After culturing, the medium is exchanged for ATX-S10·Na(II)-free fresh medium, and it is irradiated with a 670 nm laser diode at 25 J/cm². After further culturing for 48 hours, centrifugation is performed at 800 × g and the supernatant is collected.

The following method can be used to determine whether or not the malignant lymphoma or leukemia vaccine is present in the obtained supernatant. Specifically, 30 µL of the supernatant is first intradermally administered into the dorsal skin of mice once every week for 4 weeks. One week after the final administration, 1 × 10⁴ malignant lymphoma cells or leukemia cells are subcutaneously transplanted, and the mice are sacrificed on the 90th day after transplantation. Also with respect to mice to which the supernatant has not been administered, 1 × 10⁴ malignant lymphoma cells or leukemia cells are subcutaneously transplanted, and the mice are sacrificed on the 90th day after transplantation. The proliferations of malignant lymphoma cells or leukemia cells in the mice of the supernatant-administered group and the non-administered group are compared. If the proliferation of malignant lymphoma cells or leukemia cells in the mice of the supernatant-administered group is significantly greater compared to the mice of the non-administered group, it can be judged that the malignant lymphoma or leukemia vaccine is present in the supernatant.

### Examples

Examples of the invention will now be described, with the understanding that the examples are in no way limitative on the invention.

### (Example 1: Measurement of ATX-S10·Na uptake into different types of cells)

### (Preparation of ATX-S10·Na-added solution)

After labeling the compound represented by formula (II) above (hereinafter referred to as "ATX-S10·Na(II)") with ¹⁴C, the ATX-S10·Na(II) was dissolved in saline at a concentration of approximately 1 × 10⁻³ mol/L, and the solution was filtered and sterilized with a 0.22 µm filter and then diluted with 1% FBS (fetal bovine serum)-containing RPMI1640 medium to prepare a 5 × 10⁻⁵ mol/L ATX-S10·Na(II)-added solution.

### (Preparation of different types of cells)

Erythrocytes, platelets, neutrophils and lymphocytes were prepared from blood sampled from three healthy persons (designated as A, B and C).

The erythrocytes, neutrophils and lymphocytes were prepared from heparinized blood. The heparinized blood and 6% dextran-added saline were mixed in a proportion of 3:1 (v:v), and the mixture was allowed to stand at room temperature for 30 minutes. The upper layer leukocyte fraction was centrifuged with a centrifuge tube (900 rpm, 10 minutes, 4°C), and the precipitate was suspended in saline, superposed onto Ficoll-Hypaque^{®} solution and centrifuged (1600 rpm, 30 minutes, room temperature). The intermediate layer was used as the lymphocyte fraction (containing monocytes) and the precipitate was used as the neutrophil fraction. The neutrophil fraction was suspended in ice-cold 0.2% NaCl solution for hemolysis, and then an equivalent amount of ice-cold 1.6% NaCl solution was immediately added to restore isotonicity. The crude erythrocyte fraction was diluted with KRP (Krebs-Ringer phosphate buffer) and centrifuged (2000 rpm, 5 minutes, 4°C), and the buffy coat (leukocyte layer) was removed. This procedure was further repeated 4 times to yield the erythrocyte fraction.

The platelets were prepared from citric acid-treated blood. The citric acid-treated blood was centrifuged (800 rpm, 10 minutes, room temperature) to separate the PRP (platelet rich plasma), and then 1 mol/L citric acid (1/100 in volume in relation to the PRP) was added to the PRP and the mixture was centrifuged (2200 rpm, 10 minutes, room temperature). The precipitate was suspended in Tyrode-HEPES buffer (pH 7.3) and centrifuged (2200 rpm, 10 minutes, room temperature), and the precipitate was used as the platelet fraction.

As malignant lymphoma cells and leukemia cells, there were used THP-1 cells (human monocytic leukemia cells), EoL-1 cells (human eosinophilic leukemia cells), A3/KAW cells (human malignant lymphoma cells) and KG-1 cells (human acute myeloid leukemia cells). All of the cells were cultured using 10% FBS-containing RPMI1640 medium, with subculturing 1 to 2 times per week during the culturing. The THP-1 cells and EoL-1 cells were obtained from RIKEN BioResource Center, and the A3/KAW cells and KG-1 cells were obtained from Japan Health Sciences Foundation.

### (Measurement of ATX-S10·Na uptake)

After adding 400 µL of the cell suspension and 100 µL of the ATX-S10·Na(II)-added solution to each well of a 48-well microplate and mixing, the mixtures were incubated with a CO₂ incubator (37°C, 5% CO₂) or allowed to stand in a refrigerating chamber (approximately 4°C). The cells were separated immediately after addition (0 hours after addition) and 1 hour, 3 hours, 20 hours and 44 hours after addition, and ATX-S10·Na(II) uptake into the cells (including cell surface binding) was measured. The uptake with standing at 4°C corresponds to cell surface binding, and the value of the uptake with incubation at 37°C minus the uptake with standing at 4°C is presumed to correspond to the substantial ATX-S10·Na(II) uptake for each type of cell.

The cell separation and uptake measurement were performed in the following manner. Specifically, 1 mL of 1.5% BSA (bovine serum albumin)-containing PBS solution (ice-cold) was placed in a 1.5 mL tube. The suspension of cells to be separated was gently pipetted and mixed, and a 100 µL portion was taken and superposed onto the 1.5% BSA-containing PBS solution. For each well, the cell suspension (300 µL) was dispensed into three tubes (n = 3). After centrifugation (3200 rpm, 10 minutes, 4°C), the medium and 1.5% BSA-containing PBS solution were removed by suction, and then the precipitate was suspended in 100 µL of PBS(-), and 1 mL of PBS(-) was further added and mixed therewith. After additional centrifugation (3000 rpm, 10 minutes, 4°C), the supernatant was removed by suction, and the bottom of the tube, where the precipitate is present, was cut out and transferred to a counting vial. Then 4.5 mL of liquid scintillator was added and mixed therewith, and the radioactivity was measured for 5 minutes using a liquid scintillation counter. The measured radioactivity was used to calculate the ATX-S10·Na(II) uptake (pmol/10⁵ cells) into the cells.

Uptake into neutrophils was measured also with addition of N-formyl-L-methionyl-L-leucyl-L-phenylalanine (hereinafter referred to as "fMLP") (final concentration: 1 × 10⁻⁷ mol/L or 1 × 10⁻⁶ mol/L) or phorbol 12-myristate 13-acetate (hereinafter referred to as "PMA") (final concentration: 1 × 10⁻⁷ mol/L) to the cell suspension together with ATX-S10·Na(II).

Uptake into THP-1 cells was measured also with stimulation of the cells for approximately 24 hours with PMA (2 nmol/L, 5 nmol/L or 15 nmol/L) and addition of ATX-S10·Na(II) to the cell suspension immediately after removal of the PMA. THP-1 cells differentiate into macrophages upon stimulation with PMA.

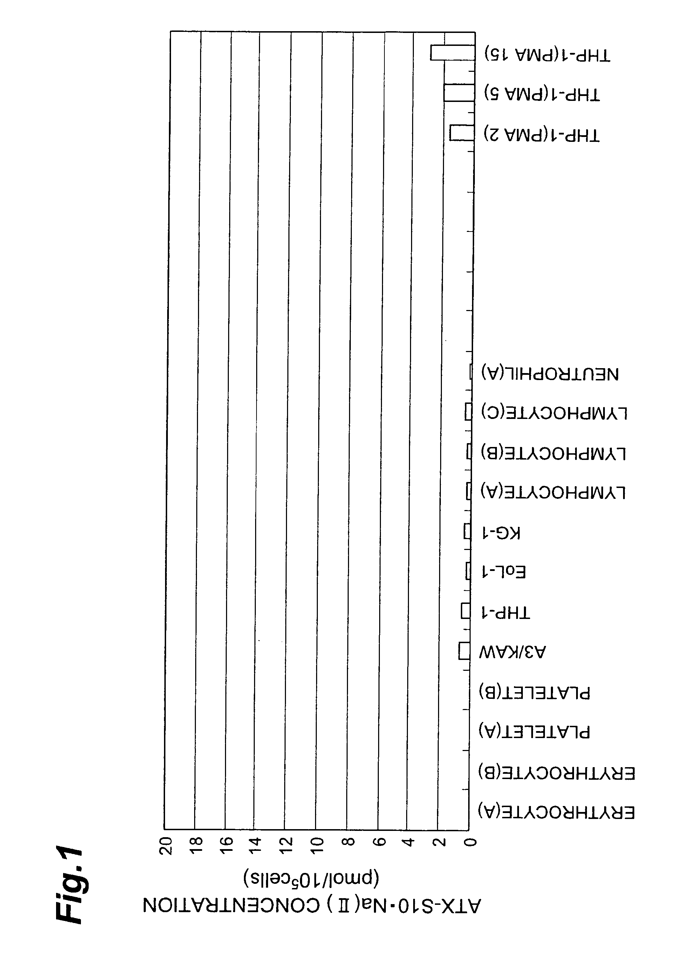

The measurement results are shown in Tables 1 to 3 and Figs. 1 to 9.

**[Table 1]**

| Time | Erythrocyte (A) | Erythrocyte (B) | Platelet (A) | Platelet (B) | Lymphocyte (A) | Lymphocyte (B) | Lymphocyte (C) |
|---|---|---|---|---|---|---|---|
| 0 hr | 0.0224 | 0.0152 | 0.0141 | 0.0048 | 0.32 | 0.31 | 0.44 |
| 1 hr | 0.0207 | 0.0255 | 0.0108 | 0.0161 | - | - | - |
| | 0.0204 | 0.0237 | 0.0107 | 0.0112 | - | - | - |
| 3 hr | 0.0211 | 0.0201 | 0.0116 | 0.0174 | 0.42 | 0.59 | 0.66 |
| | 0.0219 | 0.0224 | 0.0121 | 0.0134 | 0.33 | 0.35 | 0.42 |
| 20 hr | 0.0217 | 0.0191 | 0.0319 | 0.0435 | 1.92 | 2.30 | 2.54 |
| | 0.0180 | 0.0206 | 0.0161 | 0.0155 | 0.39 | 0.55 | 0.63 |
| 44 hr | 0.0240 | 0.0265 | 0.0695 | 0.0806 | - | - | - |
| | 0.0185 | 0.0258 | 0.0258 | 0.0389 | - | - | - |

**[Table 2]**

| Time | Neutrophil (A) | Neutrophil (B) | Neutrophil (C) | Neutrophil (fMLP10⁻⁷) | Neutrophil (fMLP10⁻⁶) | Neutrophil (PMA) |
|---|---|---|---|---|---|---|
| 0 hr | 0.21 | - | - | - | - | - |
| 1 hr | - | - | - | - | - | - |
| | - | - | - | - | - | - |
| 3 hr | 0.38 | 0.40 | 0.72 | 0.79 | 0.75 | 4.45 |
| | 0.46 | 1.27 | 0.36 | 0.97 | 2.02 | 2.76 |
| 20 hr | 4.93 | 4.04 | 10.13 | 2.67 | 3.57 | 7.33 |
| | 0.57 | 0.47 | 0.95 | 0.50 | 0.57 | 1.09 |
| 44 hr | - | 16.32 | - | - | 14.14 | - |
| | - | 0.68 | - | - | 3.28 | - |

**[Table 3]**

| Time | A3/KAW | THP-1 | EoL-1 | KG-1 | THP-1 (PMA2) | THP-1 (PMA5) | THP-1 (PMA15) |
|---|---|---|---|---|---|---|---|
| 0 hr | 0.72 | 0.56 | 0.32 | 0.40 | 1.66 | 2.07 | 2.93 |
| 1 hr | 2.38 | 3.30 | 0.25 | 2.34 | - | - | - |
| | 1.16 | 1.43 | 0.30 | 1.36 | - | - | - |
| 3 hr | 3.79 | 4.73 | 0.48 | 2.59 | 6.66 | 11.37 | 18.31 |
| | 1.06 | 2.88 | 0.32 | 1.50 | 7.05 | 8.48 | 12.74 |
| 20 hr | 10.06 | 18.39 | 1.59 | 6.85 | 11.75 | 16.06 | 19.95 |
| | 1.44 | 5.37 | 0.36 | 2.87 | 5.32 | 7.53 | 9.13 |
| 44 hr | 27.64 | 33.21 | 3.09 | 10.97 | - | - | - |
| | 5.07 | 1.94 | 0.37 | 0.96 | - | - | - |

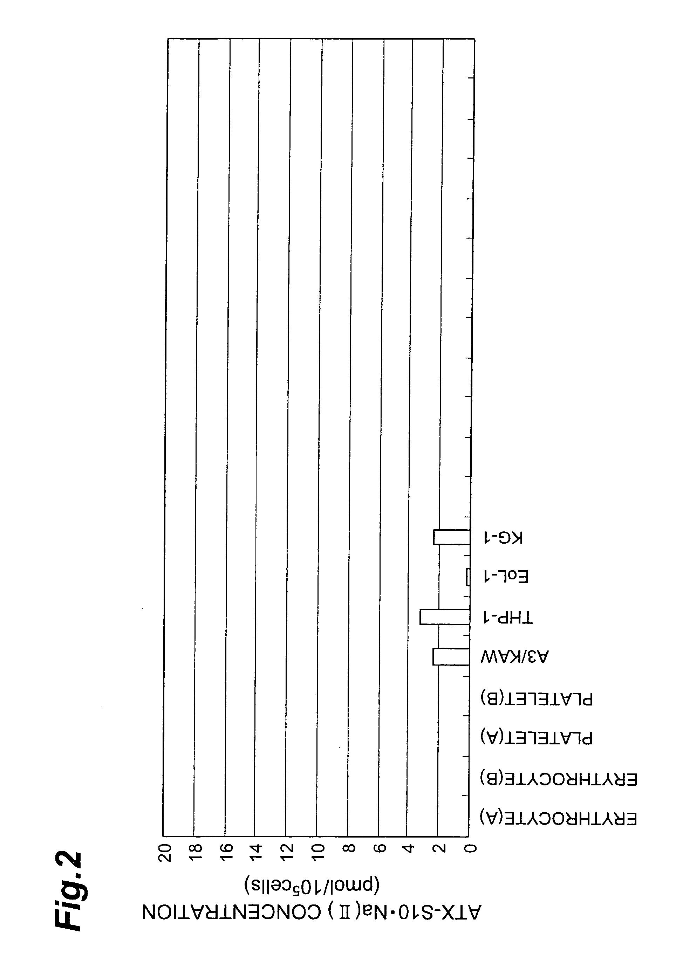

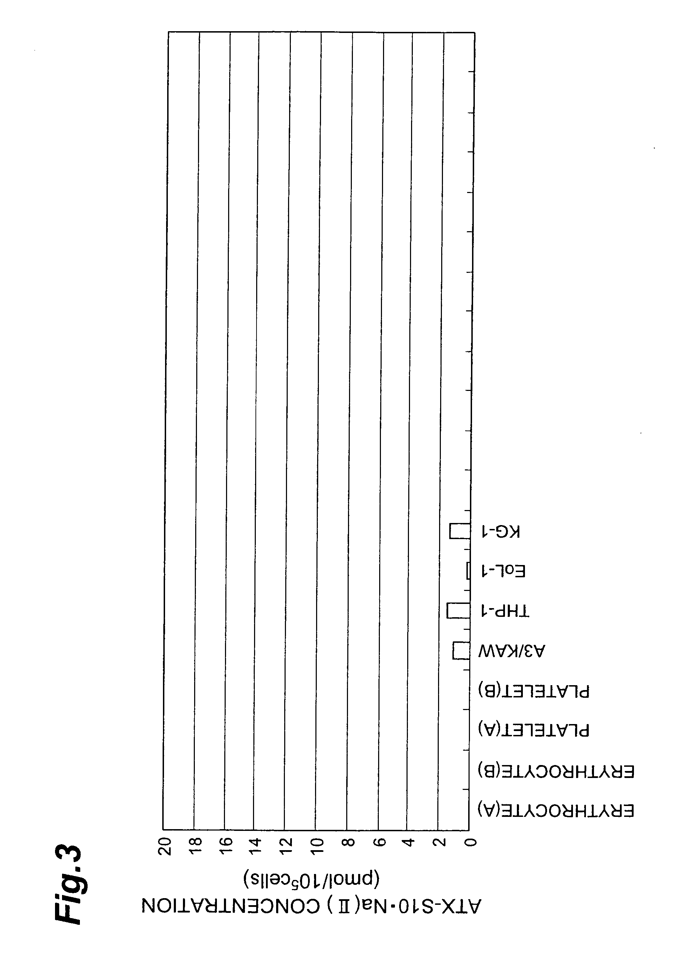

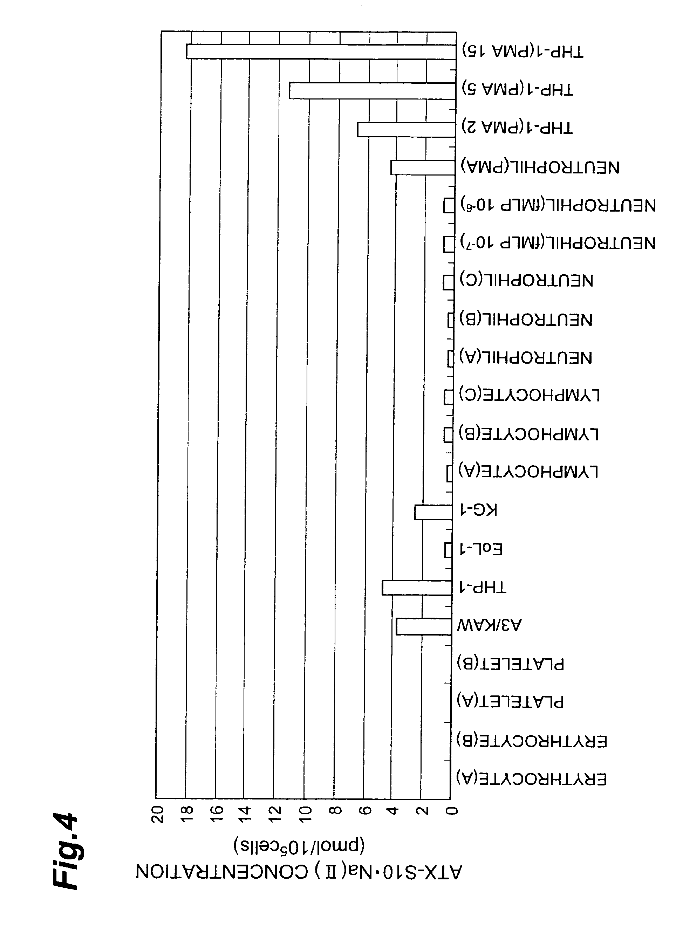

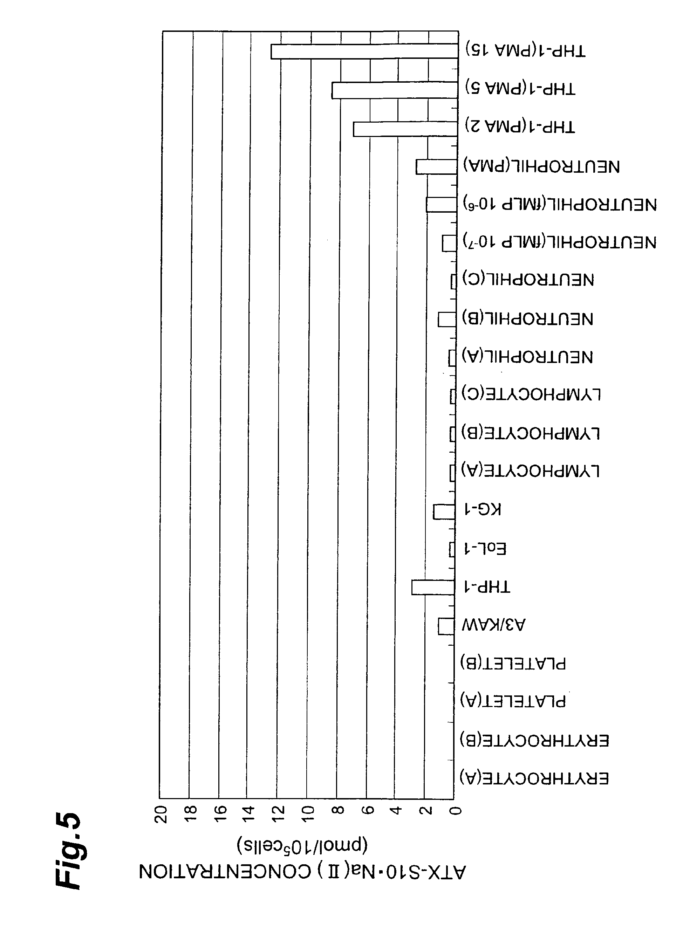

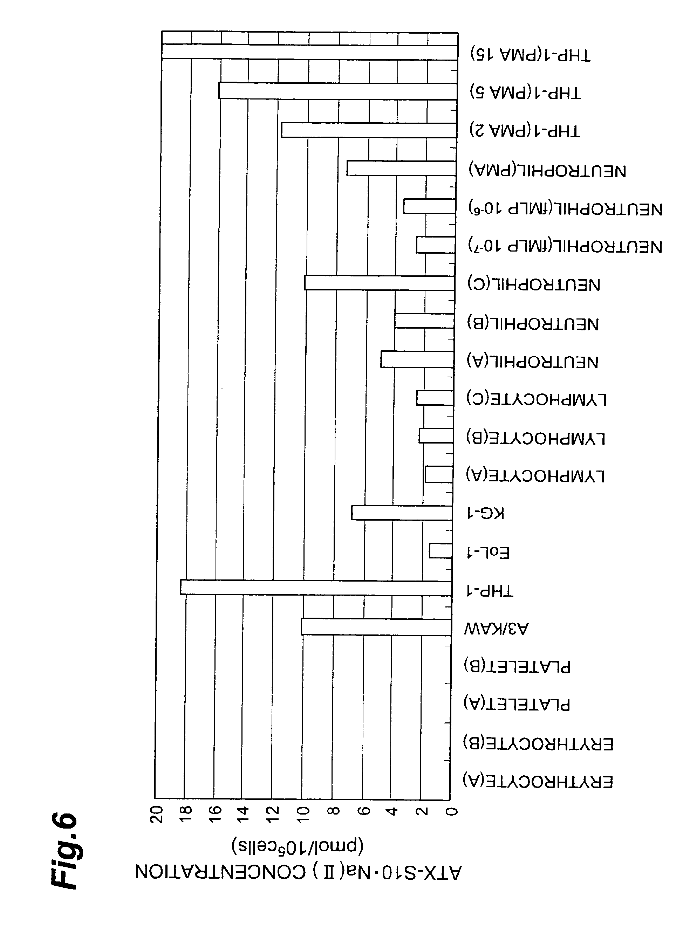

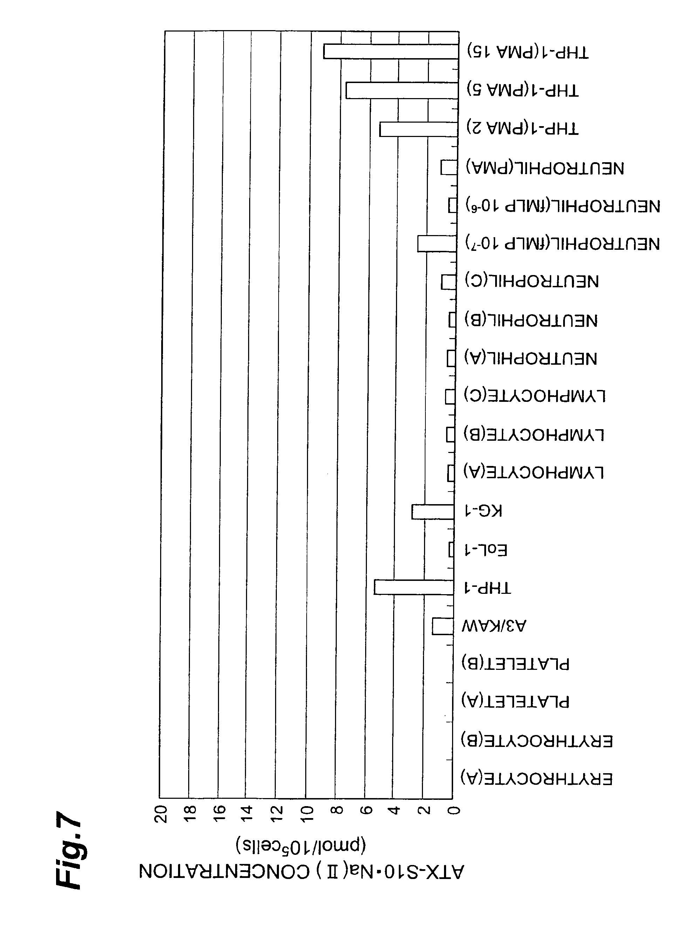

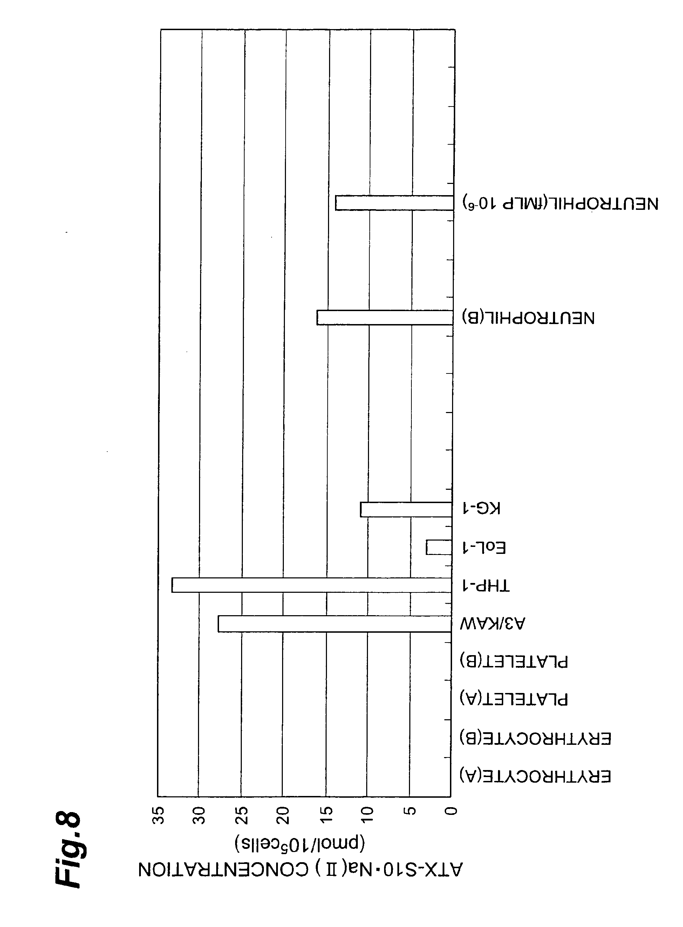

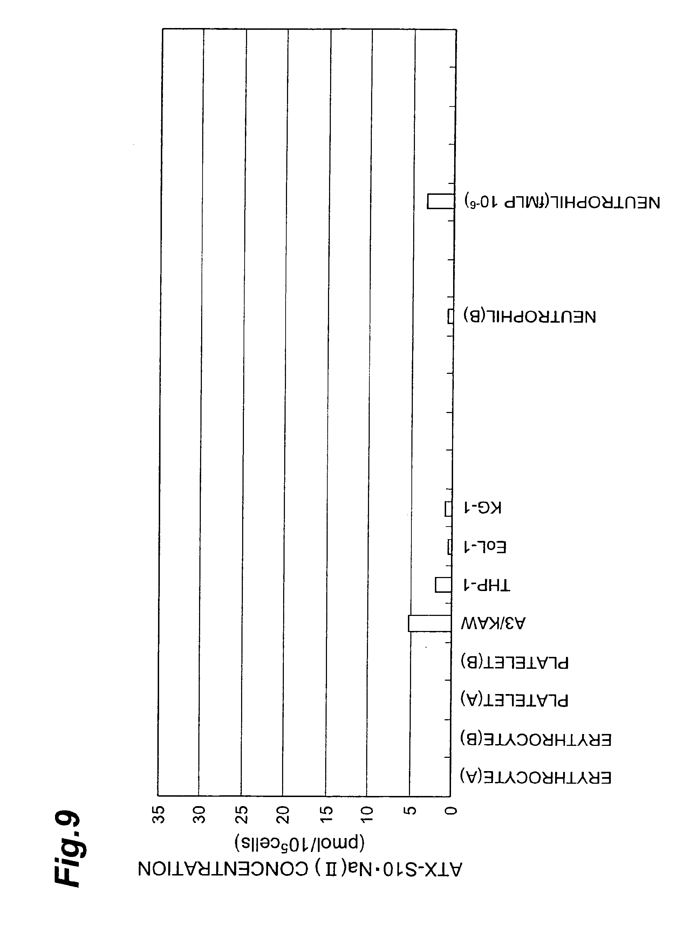

Fig. 1 is a bar graph showing ATX-S10·Na(II) uptake by different types of cells immediately after addition of ATX-S10·Na(II) to the cell suspensions. Fig. 2 is a bar graph showing ATX-S10·Na(II) uptake by different types of cells after addition of ATX-S10·Na(II) to the cell suspensions and incubation at 37°C for 1 hour. Fig. 3 is a bar graph showing ATX-S10·Na(II) uptake by different types of cells after addition of ATX-S10·Na(II) to the cell suspensions and standing at 4°C for 1 hour. Fig. 4 is a bar graph showing ATX-S10·Na(II) uptake by different types of cells after addition of ATX-S10·Na(II) to the cell suspensions and incubation at 37°C for 3 hours. Fig. 5 is a bar graph showing ATX-S10·Na(II) uptake by different types of cells after addition of ATX-S10·Na(II) to the cell suspensions and standing at 4°C for 3 hours. Fig. 6 is a bar graph showing ATX-S10·Na(II) uptake by different types of cells after addition of ATX-S10·Na(II) to the cell suspensions and incubation at 37°C for 20 hours. Fig. 7 is a bar graph showing ATX-S10·Na(II) uptake by different types of cells after addition of ATX-S10·Na(II) to the cell suspensions and standing at 4°C for 20 hours. Fig. 8 is a bar graph showing ATX-S10·Na(II) uptake by different types of cells after addition of ATX-S10·Na(II) to the cell suspensions and incubation at 37°C for 44 hours. Fig. 9 is a bar graph showing ATX-S10·Na(II) uptake by different types of cells after addition of ATX-S10·Na(II) to the cell suspensions and standing at 4°C for 44 hours.

In Tables 1 to 3 and Figs. 1 to 9, "(A)", "(B)" and "(C)" mean that the cell donors are A, B and C, respectively. "(fMLP 10⁻⁷)" and "(fMLP 10⁻⁶)" mean that the cells were stimulated with 1 × 10⁻⁷ mol/L and 1 × 10⁻⁶ mol/L fMLP, respectively, while "(PMA)" means that the cells were stimulated with 1 × 10⁻⁷ mol/L PMA. "(PMA 2)", "(PMA 5)" and "(PMA 15)" mean that the cells were stimulated with 2 nmol/L, 5 nmol/L and 15 nmol/L PMA, respectively. In Tables 1 to 3, the upper and lower numerical values for 1 hr, 3 hr, 20 hr and 44 hr represent the uptakes (pmol/10⁵ cells) with incubation at 37°C and with standing at 4°C, respectively.

As seen from Tables 1 to 3 and Figs. 1 to 9, the substantial uptake of ATX-S10·Na(II) (the value of the uptake with incubation at 37°C minus the uptake with standing at 4°C) increased with incubation time for all of the THP-1 cells, EoL-1 cells, A3/KAW cells and KG-1 cells. The uptake was highest for THP-1 cells, followed by A3/KAW cells and KG-1 cells, with a fairly low value for EoL-1 cells. In the case of THP-1 cells (activated macrophages) stimulated with PMA for 24 hours, the uptake was higher than without stimulation with PMA, and the uptake increased with higher PMA concentration.

For lymphocytes and neutrophils as well, the substantial uptake of ATX-S10·Na(II) increased with incubation time. The uptake was higher than EoL-1 cells, although not as high as THP-1 cells or A3/KAW cells. With neutrophils, there was no increase in uptake after stimulation with fMLP or PMA.

Almost no ATX-S10·Na(II) was taken up by erythrocytes and platelets. Aggregation of platelets was observed after incubation at 37°C for 44 hours.

### Industrial Applicability

The present invention can be used for treatment of malignant lymphoma, leukemia or autoimmune disease. The invention can also be used to produce a vaccine for malignant lymphoma or leukemia, and to prevent relapse and metastasis of malignant lymphoma or leukemia.

## Claims

1. A body fluid treatment method for selective *ex vivo* killing of malignant lymphoma cells, leukemia cells or activated macrophages in a body fluid, the body fluid treatment method comprising:
an addition step wherein a compound represented by formula (I) or (II) below is added to a body fluid containing malignant lymphoma cells, leukemia cells or activated macrophages that has been removed from the body, to yield an addition mixture; and
an excitation step wherein the addition mixture is irradiated with excitation light to excite the compound represented by formula (I) or (II).

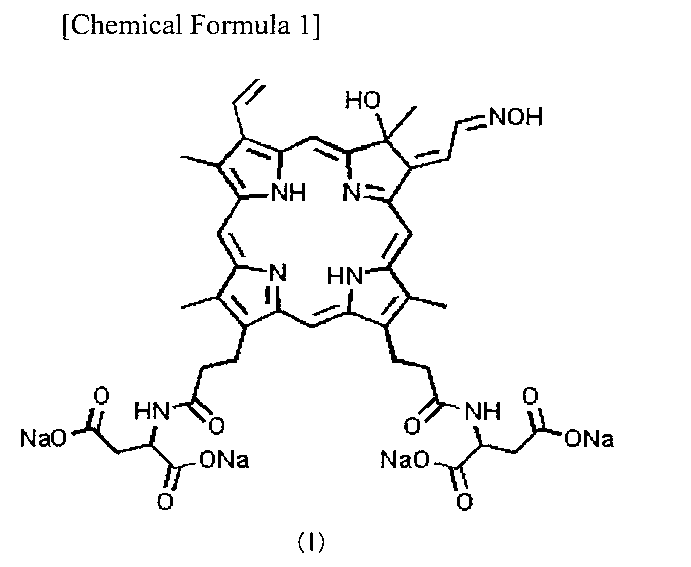

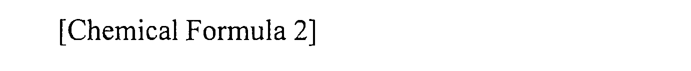

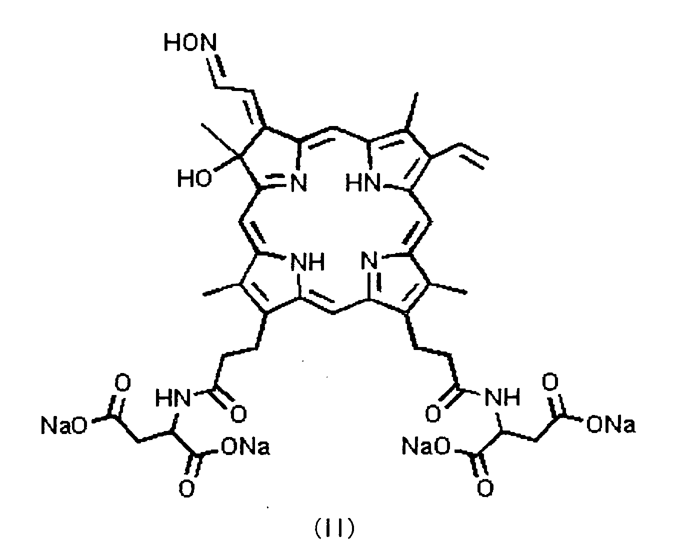

2. The body fluid treatment method according to claim 1, wherein:
prior to the addition step, there is performed a separation step in which the leukocyte fraction is separated from the body fluid containing malignant lymphoma cells, leukemia cells or activated macrophages that has been removed from the body; and
in the addition step, the compound represented by formula (I) or (II) is added to the leukocyte fraction.
